# EUROPEAN PATENT SPECIFICATION

(11) **EP 2 011 508 B1**
(45) Date of publication and mention of the grant of the patent: **22.08.2012**
(21) Application number: 07720819.7
(22) Date of filing: 16.04.2007
(51) Int. Cl.: A61K 38/16, A61K 39/085, A61P 31/04

(54) **USE OF TRAP PROTEIN PER SE AS ACTIVE INGREDIENT FOR MANUFACTURING A MEDICAMENT FOR THE TREATMENT OF STAPHYLOCOCCUS AUREUS INFECTION**
VERWENDUNG VON TRAP-PROTEIN PER SE ALS WIRKSTOFF ZUR HERSTELLUNG EINES MEDIKAMENTS ZUR BEHANDLUNG VON INFEKTIONEN MIT STAPHYLOCOCCUS AUREUS
UTILISATION DE PROTÉINE TRAP PER SE COMME PRINCIPE ACTIF POUR LA FABRICATION DE MÉDICAMENT UTILISÉ DANS LE TRAITEMENT DE L'INFECTION PAR STAPHYLOCOQUE DORÉ

(30) Priority: 17.04.2006 CN 200610075638
(43) Date of publication of application: 07.01.2009
(73) Proprietor: HAINAN GT-UNIPUL PHARMACEUTICAL CO. LTD, Xiu Ying Yong Gui Industry District Haikou Hainan 570310 (CN)
(72) Inventor: YANG, Guang, Beijing 100850 (CN); SHAO, Ningsheng, Beijing 100850 (CN); GAO, Yaping, Beijing 100850 (CN); LIU, Yu, Beijing 100850 (CN); DONG, Jie, Beijing 100850 (CN); LI, Shaohua, Beijing 100850 (CN); LIU, Chuan, Beijing 100850 (CN); SHEN, Beifen, Beijing 100850 (CN); FAN, Ming, Beijing 100850 (CN)
(74) Representative: Colombo, Stefano Paolo
(86) International application number: PCT/CN2007/001246
(87) International publication number: WO 2007/118431

(56) References cited:
- WO-A1-2005/007683
- WO-A2-2005/009396
- US-B2- 6 689 878
- YANG GUANG ET AL: "A novel peptide screened by phage display can mimic TRAP antigen epitope against Staphylococcus aureus infections", JOURNAL OF BIOLOGICAL CHEMISTRY, AMERICAN SOCIETY FOR BIOCHEMISTRY AND MOLECULAR BIOLOGY, INC, US LNKD- DOI:10.1074/JBC.M501127200, vol. 280, no. 29, 1 July 2005 (2005-07-01) , pages 27431-27435, XP002527648, ISSN: 0021-9258 [retrieved on 2005-05-19]
- BALABAN N. ET AL.: 'Regulation of staphylococcus aureus pathogenesis via target of RNAIII-activating protein (TRAP)' THE JOURNAL OF BIOLOGICAL CHEMISTRY vol. 276, no. 4, 2001, pages 2658 - 2667, XP002984048

### SEQUENCE LISTING

<110> INSTITUTE OF BASIC MEDICAL SCIENCES, ACADEMY OF MILITARY MEDICAL SCIENCES HAINAN GT-UNIPUL PHARMACEUTICAL CO.LTD.
<120> Use of TRAP protein per se as an active ingredient for the manufacture of a medicament for the treatment of Staphylococcus aureus infection
<130> IEC060027PCT
<150> 200610075638.7
   <151> 2006-04-17
<160> 4
<170> PatentIn version 3.3
<210> 1
   <211> 504
   <212> DNA
   <213> Staphylococcus aureus
<400> 1
   atgaagaaac tatatacatc ttatggcact tatggatttt tacatcagat aaaaatcaat     60
   aacccgaccc atcaactatt ccaattttca gcatcagata cttcagttat ttttgaagaa    120
   actgatggtg agactgtttt aaaatcacct tcaatatatg aagttattaa agaaattggt    180
   gaattcagtg aacatcattt ctattgtgca atcttcattc catcaacaga agatcatgca    240
   tatcaacttg aaaagaaact gattagtgta gacgataatt tcagaaactt tggtggcttt    300
   aaaagctatc gtttgttaag acctgctaaa ggtacaacat acaaaattta tttcggattt    360
   gctgatcgac atgcatacga agactttaag caatctgatg cctttaatga ccatttttca    420
   aaagacgcat taagtcatta ctttggttca agcggacaac attcaagtta ttttgaaaga    480
   tatctatacc caataaaaga atag                                            504
<210> 2
   <211> 167
   <212> PRT
   <213> Staphylococcus aureus
<400> 2
   Met Lys Lys Leu Tyr Thr Ser Tyr Gly Thr Tyr Gly Phe Leu His Gln
   1               5                   10                  15
   Ile Lys Ile Asn Asn Pro Thr His Gln Leu Phe Gln Phe Ser Ala Ser
               20                  25                  30
   Asp Thr Ser Val Ile Phe Glu Glu Thr Asp Gly Glu Thr Val Leu Lys
           35                  40                  45
   Ser Pro Ser Ile Tyr Glu Val Ile Lys Glu Ile Gly Glu Phe Ser Glu
       50                  55                  60
   His His Phe Tyr Cys Ala Ile Phe Ile Pro Ser Thr Glu Asp His Ala
   65                  70                  75                  80
   Tyr Gln Leu Glu Lys Lys Leu Ile Ser Val Asp Asp Asn Phe Arg Asn
                   85                  90                  95
   Phe Gly Gly Phe Lys Ser Tyr Arg Leu Leu Arg Pro Ala Lys Gly Thr
               100                 105                 110
   Thr Tyr Lys Ile Tyr Phe Gly Phe Ala Asp Arg His Ala Tyr Glu Asp
           115                 120                 125
   Phe Lys Gln Ser Asp Ala Phe Asn Asp His Phe Ser Lys Asp Ala Leu
       130                 135                 140
   Ser His Tyr Phe Gly Ser Ser Gly Gln His Ser Ser Tyr Phe Glu Arg
   145                 150                 155                 160
   Tyr Leu Tyr Pro Ile Lys Glu
                   165
<210> 3
   <211> 504
   <212> DNA
   <213> Staphylococcus aureus
<400> 3
   atgaagaaac tatatacatc ttatggcaca tatggatttt taaatcagat aaaaatcaat     60
   aatccatctc atcatttatt ccaattttca actgctgatt catcggtgat ttttgaagag     120
   actgaagaaa aaactgtact gaaatcacct tcaatatatg aagttattaa agaaataggc    180
   gcattcaatg aagaccattt ttattgcgca atttttattc cgtcaactga agaccatgtt    240
   tatcaacttg aaaagaaatt aatcagtgtt gatgacaatt tcaaaaactt tggtggcttt    300
   aaaagttatc gattgctgag acctgttaaa ggtacaactt acaaaattta ctttggcttt    360
   gctgatcgac aaacttatga agactttaaa aattctgatg cttttaaaga tcatttttca    420
   aaagaagcat taagtcatta ctttggttca agcggacaac attcaagtta ttttgaaaga    480
   tatttatacc caataaaaga atag                                           504
<210> 4
   <211> 167
   <212> PRT
   <213> Staphylococcus aureus
<400> 4
   Met Lys Lys Leu Tyr Thr Ser Tyr Gly Thr Tyr Gly Phe Leu Asn Gln
   1               5                   10                  15
   Ile Lys Ile Asn Asn Pro Ser His His Leu Phe Gln Phe Ser Thr Ala
               20                  25                  30
   Asp Ser Ser Val Ile Phe Glu Glu Thr Glu Glu Lys Thr Val Leu Lys
           35                  40                  45
   Ser Pro Ser Ile Tyr Glu Val Ile Lys Glu Ile Gly Ala Phe Asn Glu
       50                  55                  60
   Asp His Phe Tyr Cys Ala Ile Phe Ile Pro Ser Thr Glu Asp His Val
   65                  70                  75                  80
   Tyr Gln Leu Glu Lys Lys Leu Ile Ser Val Asp Asp Asn Phe Lys Asn
                   85                  90                  95
   Phe Gly Gly Phe Lys Ser Tyr Arg Leu Leu Arg Pro Val Lys Gly Thr
               100                 105                 110
   Thr Tyr Lys Ile Tyr Phe Gly Phe Ala Asp Arg Gln Thr Tyr Glu Asp
           115                 120                 125
   Phe Lys Asn Ser Asp Ala Phe Lys Asp His Phe Ser Lys Glu Ala Leu
       130                 135                 140
   Ser His Tyr Phe Gly Ser Ser Gly Gln His Ser Ser Tyr Phe Glu Arg
   145                 150                 155                 160
   Tyr Leu Tyr Pro Ile Lys Glu
                   165

## Description

### Technical Field

The present invention belongs to the biological pharmacy field. Specifically, the present invention relates to the use of TRAP protein per se as an active ingredient for the manufacture of a medicament for the treatment of *Staphylococcus aureus* infection.

### Technical Art

*Staphylococcus aureus* belongs to *Staphylococcus* and is a major pathogenic bacterium that causes severe infections of pneumonia, endocarditis, burn and war wound, septicemia, and toxic shock, etc. In addition, *Staphylococcus aureus* is one kind of bacterium that causes food-poisoning. *Staphylococcus aureus* widely exists in air, soil, water and feeding utensils, and animal and human beings are main vectors. *Staphylococcus aureus* can be transmitted through spit and contact on a large scale in anthills. There are more than millions of patients suffering with *Staphylococcus aureus* infections only in hospital per year. *Staphylococcus aureus* is a major pathogenic bacterium that causes Gram-positive sepsis, infections of burn wound and acute hepatic failure.

The main pathogenic substances of *Staphylococcus aureus* are exotoxins, including hematoxin, lueocidin, enterotoxin, etc. The recent researches indicate that the synthesis of these virulence factors in *Staphylococcus aureus* is controlled by a regulatable RNA molecule, i.e., RNA III. RNAIII activates the gene transcription of virulence factors and regulates the translation of virulence factors by base complementarity. The RNAIII level is relatively lower during the early log phase of bacterial growth, but increases 40 times during the late log phase of bacterial growth, while the level of RNA III is regulated by the protein RAP (RNA III activating protein) secreted by *Staphylococcus aureus* per se, so that RAP is also called as *Staphylococcus aureus* virulence stimulating factor. *Staphylococcus aureus* continuously secretes RAP, and RAP activates the production of virulenece factors only when RAP reaches a certain concentration. *Staphylococcus aureus* without the production of RAP does not cause diseases (Balaban N. et al, Science 1995, 280, 438-440). The recent researches show that RAP activates the transcription of RNA III through the mediation of a 21 KD protein TRAP (Target of RNA III-activating protein). When the gene encoding TRAP is inactivated by mutation, RAP cannot activate the transcription of RNAIII.

TRAP consists of 167 amino acids and has His kinase activity. TRAP is phosphorylated during the early phase of the growth of *Staphylococcus aureus,* and reaches the maximum level during the middle log growth phase. After the RAP action, the signal transduction is performed by autophosphorylation, thereby increasing the intracellular level of RNAIII and accelerating the secretion of *Staphylococcus aureus* exotoxins (Namomi B. et al, J. Biol. Chem., 2001, 276: 2658-2667). TRAP is a surface protein of *Staphylococcus aureus* and is highly conserved in *Staphylococcus.* The main biological function of TRAP is to participate in the regulation of *Staphylococcus aureus* exotoxins. The level of *Staphylococcus aureus* exotoxins is up-regulated with the increase of phosphorylation level of TRAP (Gov Y et al , J. Biol. Chem., 2004, 279: 14665-72).

Based on sequence alignment analysis, it has been found at present that TRAP proteins can be divided into three families and they have a homology more than 80% between each other (Yang G. et al, J. Biol. Chem., 2005, 280: 27431-27435). Specifically, it has been found in accordance with the sequence alignment, TRAP proteins are highly conserved in *Staphylococcus aureus* and *Staphylococcus epidermidis.* At present, there are TRAP sequences of 10 *Staphylococcus aureus* strains and 2 *Staphylococcus epidermidis* strains logged in Gene Bank.

TRAP proteins are divided into following 3 families on the basis of sequence conservation: Family 1 includes 7 TRPA proteins from *Staphylococcus aureus* which have an identity of 99.49% based on the analysis by DNAMAN (Figure 1); Family 2 includes 3 TRPA proteins from *Staphylococcus aureus* which have an identity of 99.80% based on the analysis by DNAMAN (Figure 2); and Family 3 includes 2 TRAP proteins from *Staphylococcus epidermidis* which have an identity of 98.20% (Figure 3). In addition, the TRAP proteins from *Staphylococcus aureus* RN6390B, *Staphylococcus aureus* 04018 and *Staphylococcus epidermidis* ATCC12228 in said three families have an identity of 84.72 % based on the sequence alignment analysis by DNAMAN (Figure 4).

The recent researches show that the pathogenic effect of *Staphylococcus aureus* can be effectively reduced by inhibiting the pathways for the production of *Staphylococcus aureus* exotoxins by using exogenous RAP and TRAP inhibitors. The known RAP and TRAP inhibitors such as small molecular inhibiting peptides (RNAS III inhibiting peptide, hereafter called RIP) and antibodies can effectively reduce the secretion of *Staphylococcus aureus* exotoxins. The C-terminal of TRAP can be used as an effective peptide vaccine to protect organisms from the *Staphylococcus aureus* infections (Oleny V. et al, Peptides, 2001, 22: 1621-1627; Yang G. et al, Peptides, 2003, 24, 1823-1828; Yang G. et al, J. Biol. Chem., 2005, 280: 27431-27435).

At present, the *Staphylococcus aureus* infections are mainly treated by inhibiting the pathways for the production of *Staphylococcus aureus* toxins described above to reduce the production of *Staphylococcus aureus* toxin, such as via RIP, RAP antibodies and the like, thereby reducing the pathogenic effect of *Staphylococcus aureus.*

The DNA sequence and amino acid sequence of a target of RNAIII-activating protein (TRAP) from *Staphylococcus aureus* RN6390B is known in the art (USP6689878). In addition, it has been reported that said TRAP is used to induce the production of an antibody and said antibody can be used as a medicament (USP6747129), wherein function of TRAP is inhibited by monoclonal or polyclonal antibodies against TRAP to inhibit the occurrence of *Staphylococcus aureus* infections.

On the one hand, the drug resistance of pathogenic bacteria including *Staphylococcus aureus* is continuously generated and increased due to the abuse of antibiotics, and many antibiotics are ineffective to the drug resistant strains, including Methicillin resistant S.aureus (MRSA) that is resistant to β-lactam antibiotics, glycolpeptide resistant S.aureus (GISA) that is resistant to glycolpeptide antibiotics, and vancomycin resistant S.aureus (VRSA), etc. On the other hand, *Staphylococcus aureus* per se readily develop drug resistance.

To solve the above problems existing in the treatment of *Staphylococcus aureus* infections at present, it is urgent to develop a method that can effectively control the *Staphylococcus aureus* infections but cannot make *Staphylococcus aureus* readily develops drug resistance.

### Invention contents

On one aspect, the present invention relates to the use of TRAP per se as an active ingredient for the manufacture of a medicament for the treatment of *Staphylococcus aureus* infection, wherein TRAP has a sequence as defined in the claims.

Specifically, it is surprisedly found, from an in vitro test (MDBK cell model) and in vivo test (animal model of *Staphylococcus aureus* infection), that an exogenous TRAP per se can effectively inhibit the production of *Staphylococcus aureus* exotoxins in the cell model and animal model. The exogenous TRAP per se can be used as an active ingredient to treat *Staphylococcus aureus* infections and reduce the pathopoiesis, and its therapeutic effect is similar to that of polyclonal antibodies against TRAP.

In the present invention, the term "exogenous TRAP" refers to the TRAP proteins added or administrated artificially for the treatment of *Staphylococcus aureus* infections with respect to endogenous TRAPs produced in said cell or animal model of *Staphylococcus aureus* infection during the production of *Staphylococcus aureus* exotoxins after *Staphylococcus aureus* infection. In the present invention, as to an isolated TRAP that is extracted by manual work from *Staphylococcus aureus* or recombinantly expressed by genetic engineering, whether it directly come from a specific *Staphylococcus aureus* strain causing *Staphylococcus aureus* infections or a strain having a high homology thereto, if only said isolated TRPA is additionally added or administrated for the treatment of the *Staphylococcus aureus* infection, it is recognized as an exogenous TRAP.

Specifically, in one embodiment of the present invention, MDBK cell model is employed, and an exogenous TRAP is or is not added to the MDBK cell culture liquid at the time when *Staphylococcus aureus* is added to said liquid. The results show that the production of *Staphylococcus aureus* exotoxins can be effectively inhibited by the addition of an exogenous TRAP. In another embodiment of the present invention, after *Staphylococcus aureus* is peritoneally injected to mice, then an exogenous TRAP protein is or is not injected, and the survival time and status of each group of mice are observed.

The results show that the *Staphylococcus aureus* infection in mice is reduced by the injection of an exogenous TRAP, thereby increasing the survival rate of mice.

In the present invention, said TRAP proteins include native TRAP proteins from different kinds of *Staphylococcus aureus,* recombinant TRAP proteins or fusion TRAP proteins prepared by such as a gene engineering method, and TRAP proteins and/or chemically modified TRAP protein derivatives obtained by such as a chemical synthesis method. The TRAP proteins of the present invention also include biologically active fragments derived from an intact TRAP protein which retain fully or partially biological active. Said fragments of TRAP proteins can also be used to treat *Staphylococcus aureus* infections.

In USP6689878 and USP6747129 the amino acid sequences of TRAP proteins and the polynucleotide sequences encoding them are disclosed in these documents.

In the present application, said TRAP proteins refer to the proteins have a sequence identity to SEQ ID NO: 2 or SEQ ID NO: 4, or the orthologs thereof, including the proteins that have a sequence identity of at least 70%, at least 80%, at least 95%, at least 98% or 100% thereto.

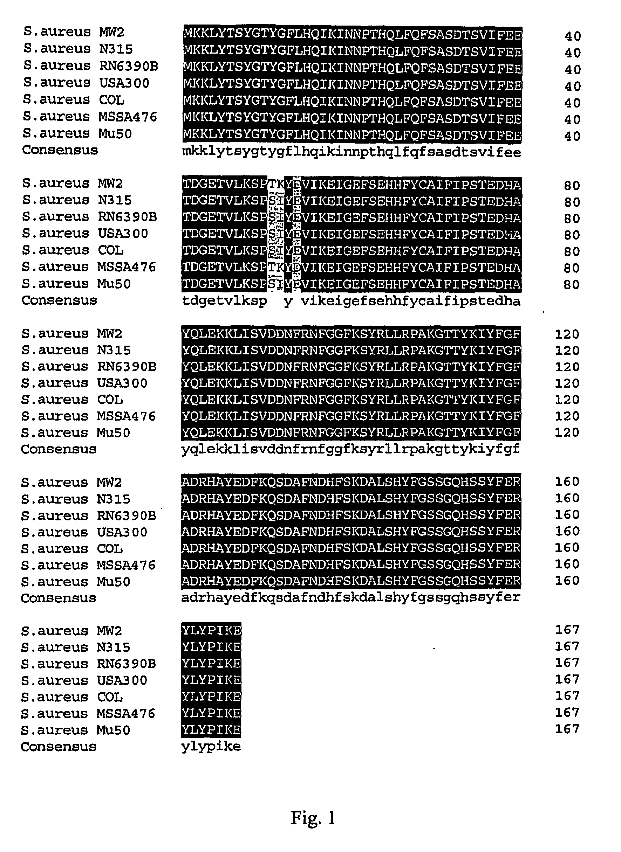

In one embodiment of the present invention, said TRAP protein is selected from the sequences as shown in SEQ ID NO: 2, SEQ ID NO: 4, and the TRAP proteins from 12 different origins belonging to 3 different protein families as shown in Figures 1-3.

The identity percentage between two amino acid sequences can be determined by such as Needelman-Wunsch alignment which can be used in the alignment of protein and DNA. As to the protein alignment, the default scoring matrix used is BLOSUM50 in which the penalty of one residue in spacer is -12, while that of other residues is -2, and the alignment can be carried out by FASTA package, an alignment software of Version v20u6 (W.R.Pearson and D.J.Lipman, "Improved tools for Biological Sequence Analysis", PNAS, 2005, 85: 2444-2448; and W.R.Pearson, "Rapid and Sensitive Sequence Comparison using FASTP and FASTA", Methods in Enzymology, 1990, 183: 63-98).

The TRAP proteins of the present invention may be selected from any known TRAP proteins. With respect to the structure and origin, said TRAP proteins may be the same as or different from the endogenous TRAP proteins produced by *Staphylococcus aureus* that causes *Staphylococcus aureus* infection to be treated. The inventors discover that the TRAP proteins from multiple kinds of different *Staphylococcus aureus* strains have the same or similar effects on the inhibition of the production of *Staphylococcus aureus* exotoxins and the reduction of pathogenicity of *Staphylococcus aureus.*

In one embodiment of the present invention, the TRAP protein as shown in SEQ ID NO: 2 from *Staphylococcus aureus* RN6390B (which corresponds to the nucleotide sequence AF202641 in GenBank^{™}) is employed as an active ingredient to effectively inhibit the production of *Staphylococcus aureus* exotoxins in an in vitro test and in vivo test. In another embodiment, the TRAP protein as shown in SEQ ID NO: 4 from *Staphylococcus aureus* 04018 (which corresponds to the nucleotide sequence AY248703 in GenBank^{™}) as an active ingredient to inhibit the production of *Staphylococcus aureus* exotoxins in an in vitro test and in vivo test, thereby treating *Staphylococcus aureus* infections. In another embodiment of the present invention, a fusion protein consisting of TRAP protein and a polypeptide of 12 amino acids is used to inhibit the productionof *Staphylococcus aureus* exotoxins in an in vitro test and in vivo test, thereby treating *Staphylococcus aureus* infections.

Also described in the application is a pharmaceutical composition comprising an above said TRAP protein as an active ingredient and an optionally pharmaceutically acceptable carrier or excipient. According to the present invention, the TRAP proteins or fragments thereof for the treatment of *Staphylococcus aureus* infections may be administrated alone in a single dose or multiple doses, or is administrated in combination with a pharmaceutically acceptable carrier or excipient. The pharmaceutical composition comprising a TRAP protein or an active fragment thereof for the treatment of *Staphylococcus aureus* infections in the present invention can be formulated together with a pharmaceutically acceptable carrier or diluent and any known adjuvant or excipient by the traditional techniques as disclosed in the book (Remongton, Science and Practice of Pharmacology, 19th edition, Genearo, Ed., Mack Piblishing Co., Easton, PA, 1995), and is in a traditional form such as solution, suspension, freeze-dried powder, capsule, tablet or aerosol, etc.

The pharmaceutical composition can be specifically formulated to be administrated in any suitable route, such as parenteral (which includes subcutaeous, intramuscular, intrathecal, intravenous and intradermal), oral, rectal, nasal, pulmonary, topical (which includes buccal and subglossal), curtaneous, intracisternal, endoperitoneal and vaginal routes. It should be understood that the preferred route depends on the general conlition and age of an object who accepts the treatment, the kind of symptom to be treated, and the active ingredient used. The administration route may be any one of routes, if only the active ingredient can be effectively transported to suitable or expected action sites by such route.

The pharmaceutical composition for parenteral administration includes sterile aqueous or non-aqueous injectable solutions, dispersions, suspensions or emulsions, and sterile powders or freeze-dried powders, which are reconstructed in a sterile solution for injection or dispersion before they are used. The storage formulations for injection are also within this scope of the present invention.

The pharmaceutical composition for oral administration includes solid dosage form, such as hard or soft capsules, tablets, troches, sugar-coated pills, pills, lozenges, powders or granules. If suitable, they can be provided with a coating, such as enteric coating, or formulated by any the known techniques in the art to control the release of active ingredient, such as sustained release or prolonged release.

The liquid dosage form for oral administration includes solutions, emulsions, aqueous or oily suspensions, syrups and elixirs.

Other suitable dosage forms including suppositories, aerosols, ointments, creams, gelatins, inhalations, pasters for skin, and implants, etc.

The typical administration dosage is in a range from about 0.001 to about 100 mg per kg weight per day, such as from about 0.01 to about 50 mg per kg weight per day such as from about 0.05 to 10 mg per kg weight are administrated per day, in one or more doses such as 1 to 3 doses. The exact dosage depends on the status of *Staphylococcus aureus* infections to be treated, administration frequency and way, the gender, age and general condition of an object who accepts the treatment, the kind and severity of the symptom to be treated, any concomitant disease to be treated and other factors known by a person skilled in the art in the art.

The formulation may expediently exhibit a unit-dose form by a method known in the art. The formulation for parenteral administration, such as intrathecal, intravenous and intramuscular administration and the like is administrated at one or more times a day (such as once to trice a day) in a typical dosage ranging from about 0.001 to about 1000 mg, for example, from about 0.01 to about 500 mg such as from about 0.1 mg to about 100 mg.

The formulation for oral administration is administrated at one or more times a day (such as once to trice a day) in a typical dosage ranging from about 0.05 to about 1000 mg, for example, from about 0.1 to about 500 mg such as from about 0.5 mg to about 200 mg.

The suitable pharmaceutically acceptable carrier includes inert solid diluents or fillers, sterile aqueous solution and multiple organic solvents. For example, the solid carriers include lactose, gypsum powder, sucrose, cyclodextrin, talcum powder, gelatin, agar, pectin, gum Arabic, magnesium stearate, stearic acid and cellulose lower alkyl ether. Examples of liquid carriers are syrup, peanut oil, olive oil, phospholipids, fatty acid, fatty acid amide, polyoxyethelene and water. Similarly, carriers or diluents may include any sustained release substance known in the art, such as monostearin or distearin, which is used alone or in combination with a wax. The pharmaceutical composition of the present invention that is obtained by the combination of a TRAP protein or a fragment thereof with a pharmaceutically acceptable carrier and used for the treatment of *Staphylococcus aureus* infections may be expediently administrated by any disclosed administration routes in a multiple-dose form. The formulation may expediently exhibit a unit-dose form by a method known in the art.

The aqueous suspensions may comprise a TRAP protein or a fragment thereof which is mixed with an excipient suitable for the production of an aqueous suspension. The excipient is a suspending agent, such as carboxymethyl cellulose sodium, methylcellulose, hydroxypropyl methylcellulose, sodium alginate, polyvinylpyrrolidone, bassora gum and gum Arabic; dispersing or wetting agent may be native phospholipids, such as lecithin, or a condensation product of an alkylene oxide and a fatty acid, such as polyoxyethylene stearate, or a condensation product of ethylene oxide and a long chain aliphatic alcohol such as heptadecanylethyleneoxyhexadecanol, or a condensation product of ethylene oxide and a partial ester derived from fatty acid and hexitol, such as polyoxyethylene sorbitol monooleate, or a condensation product of ethylene oxide and a partial ester derived from fatty acid and hexitol glycoside, such as polyoxyethylene sorbitan monooleate. The aqueous suspension may further comprise one or more toners, one or more flavoring agents and one or more sweetening agents such as sucrose or glucide.

The oily suspension may be prepared by dispersing an active ingredient to vegetable oil (such as peanut oil, olive oil, sesame oil or cocoanut oil) or a mineral oil (such as liquid paraffin). The oily suspension may comprise a thickening agent such as beeswax, hard paraffin or spermol. The above sweetening agents and flavoring agents may be added to obtain a palatable oral formulation. In addition, an antioxidant (such as ascorbic acid) may be added to the compositions for preservation.

The dispersible powders or granules suitable for preparing an aqueous suspension by adding water are obtained by mixing an active ingredient, dispersing or wetting agent, suspending agent and one or more preservatives. The above mentioned dispersing or wetting agent may be used as examples of suitable dispersing or wetting agent. Other excipients, toners, flavoring agents and sweetening agents may be added to the suspension.

According to the present invention, the pharmaceutical composition comprising a TRAP protein or a fragment thereof for the treatment of *Staphylococcus aureus* infections may be present in an oil in water emusion, wherein the oil phase may be vegetable oil (such as olive oil or peanut oil), or a mineral oil (such as liquid paraffin), or a mixture thereof. The suitable emulsifying agent may be a native gum such as gum Arabic, bassora gum, a native phospholipid such as soya and lecithin, an ester or partial ester derived from a fatty acid and hexitol glycoside such as sorbitan monooleate, and a condensation product of said partial ester and ethylene oxide such as polyoxyethylene sorbitan monooleate. The emulsifying agent may comprise sweetening agents and flavoring agents.

The composition may be in present in a suppository form for rectal administration. This composition may be prepared by mixing drugs and a suitable non-irritative excipient, wherein the excipient is a solid at general temperature but is liquefied at rectal temperature, so that it can be thawed in recta to release drugs. Such excipient includes cocoa butter and polyethylene glycol, etc.

As to the external use, the creams, ointments, jellys and suspensions comprising the compound of the present invention are contemplated. For this application purpose, the dentilaves and gargarismas are also comprised therein.

According to the present invention, TRAP proteins or fragments thereof for the treatment of *Staphylococcus aureus* infections may be administrated through a liposome delivery system, such as small monolayer vesicles, big monolayer vesicles or multilaminar vesicles, wherein the liposome may consist of different phosphatides such as cholesterol, stearyl amine or phosphatidyl choline.

In addition, according to the present invention, part of TRAP proteins or fragments thereof for the treatment of *Staphylococcus aureus* infections may be mixed with water or a general organic solvent to form solvates. Such solvates are involved in the scope of the present invention.

If a solid carrier is for oral administration, the formulation may be in form of a tablet, a hard gelatin capsule in which drugs in a powder or pellet form are placed into, a troche or a lozenge. The amount of a solid carrier varies greatly, but it is generally from about 25 mg to about 1 g. If a liquid carrier is used, the formulation may be in form of a syrup, emulsion, soft capsule of geltin or sterile injectable liquid, such as aqueous or non-aqueous liquid suspensions or solutions.

According to the present invention, TRAP proteins or active fragments thereof for the treatment of *Staphylococcus aureus* infections may be administrated to a mammalian in need of such treatment, in particular a human being. Said mammalian also includes animals, whether they are tamed ones (e.g. domestic pets) or non-tamed ones (e.g. wild animals).

The pharmaceutical composition comprising a compound as described herein may be administrated at once or more times a day or a week. The effective amount of the pharmaceutical composition is one that arrives at the clinically significant effect. Such dosage partially depends on the specific symptom needed to be treated, the age, weight and general health conition of a subject and other factors obviously considered by a person skilled in the art.

The term "treatment" herein refers to the treatment or nursing of a patient to help him against *Staphylococcus aureus* infections. This term aims to cover the entire scope of the treatment of *Staphylococcus aureus* infections including the alleviation and abatement of symptoms and complications, and/or cure or elimination of diseases, disorders and symptoms. The subjects who accept such treatment are preferably mammalian, in particular, human beings.

In the present invention, the term "therapeutically effective amount" refers to an amount sufficient for the treatment of status of said diseases, and it can vary based on a patient, a disease to be treated and a treatment method used. As to bacterial infections, especially *Staphylococcus aureus* infections, said therapeutically effective amount refers to an amount sufficient to successfully prevent or eliminate said infections after the occurrence of infections.

According to the present invention, TRAP proteins or biological active fragments thereof for the treatment of *Staphylococcus aureus* infections may be administrated alone or in combination with other established therapeutic methods.

It is discovered in the present invention that TRAP has double effects against *Staphylococcus aureus* infections, i.e., the protein per se and an inhibitor thereof or a neutralizing antibody thereof have the same effects, which will represent a kind of novel action mode of medicaments.

It is also discovered in the present invention when TRAP is used to treat *Staphylococcus aureus* infections, it certainly induces the production of antibodies against TRAP in a human body, because TRAP is an exogenous protein which is from bacteria. As to a traditional medicament, the production of antibodies means the inhibition of the effects of medicament. However, as to TRAP, the production of antibodies is useful.

The advantages of the present invention are as follows.
(1) The novel use of TRAP in the manufacture of a medicament is discovered, and a novel field relating to the use of a protein associated with bacterial cell membranes for preparing therapeutic medicament is developed, which represents a new medicament mode.
(2) No homologous sequence of the TRAP proteins of the present invention is found in human genome, so that the medicaments of TRAP cannot cause human immunologic diseases.
(3) TRAP also stimulates a body to produce corresponding antibodies at the time when it inhibits the production of *Staphylococcus aureus* exotoxins, and the produced antibodies can further inhibit the generation of *Staphylococcus aureus* exotoxins. The mode that TRAP firstly effects and then the produced antibodies further play the same role provides a novel medicament action mode.
(4) When a medicament comprising a TRAP protein is used to treat *Staphylococcus aureus* infections, since it mainly inhibits the production of exotoxins to reduce the pathogenicity of *Staphylococcus aureus,* it does not affect the survival of bacteria and it is not easy to cause the generation of drug resistance. Thus, the medicament can also effect on the drug-resistant staphylococci.

### Brief description of the drawings

Fig.1: the sequence alignment results of 7 kinds of TRAP proteins from different origins in TRAP family 1

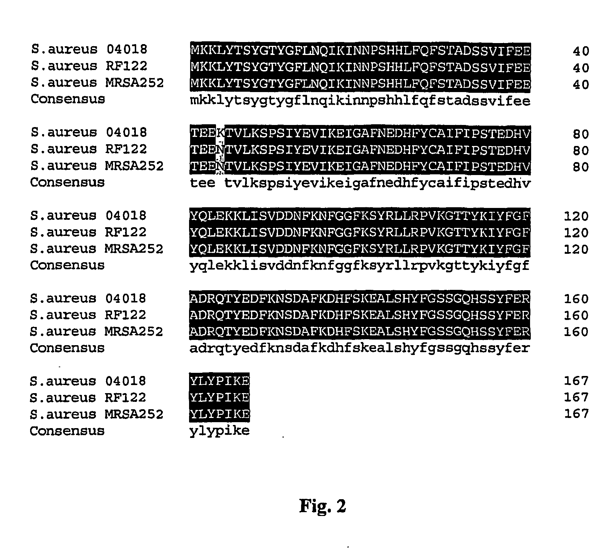

Fig.2: the sequence alignment results of 3 kinds of TRAP proteins from different origins in TRAP family 2

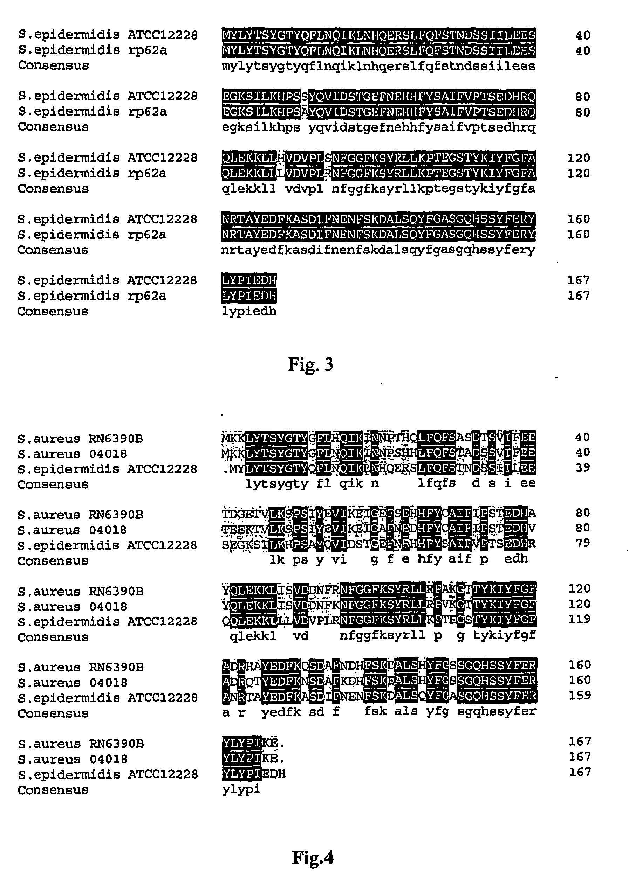

Fig.3: the sequence alignment results of 2 kinds of TRAP proteins from different origins in TRAP family 3
Fig.4: the sequence alignment results of TRAP proteins from three different strains in three TRAP families

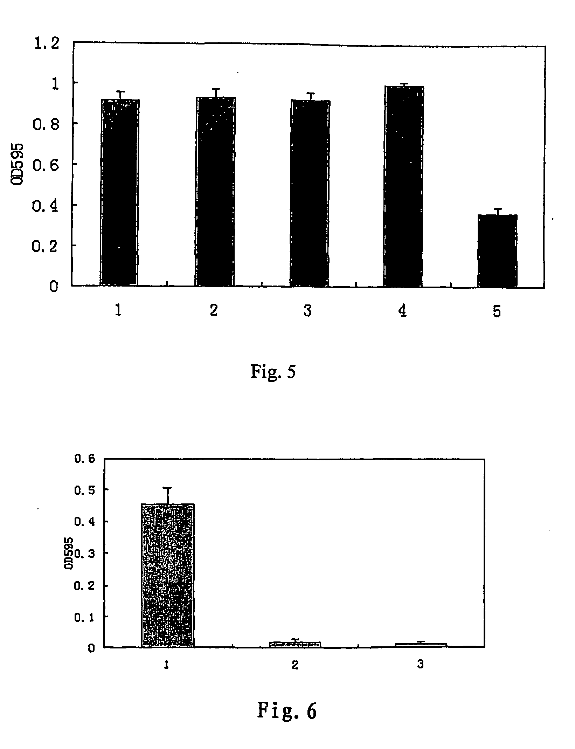

Fig.5: the histogram of the effect of exogenous proteins from different origins on the production of *Staphylococcus aureus* exotoxins, wherein the numerals respectively represent the origins of TRAP, i.e., 1 represents the proteome with GenBank^{™} Accession No.: AF202641; 2 represents the proteome with GenBank^{™} Accession No.: AY248703; 3 represents the native TRAP proteome purified from *Staphylococcus aureus;* 4 represents fusion pretein consisting of TRAP pretein and a polyeptide of 12 aminacids; 5 represents normal cells control; and 6 presents physiological saline control group.
Fig.6: the histogram of the effects of exogenous TRAP proteins on the productions of antibodies against TRAP, wherein 1 represents the group treated with TRAP; 2 represents the saline control group; and 3 represents the negative control.

### Specific modes for carrying out the invention

The present application is further illustrated in detail by the following several examples, but they do not limit the present invention in any form.

### Preparation example 1

The preparation of two TRPA proteins of *Staphylococcus aureus* family (TRAP proteins (GenBank^{™} Accession Nos.: AF202641 and AY248703)). Two TRAP proteins were separately obtained by designing primers in accordance with base sequence in Genbank by a method disclosed by Yang G. et al (2005), amplifying TRAP gene from genome of strain RN6390B or 04018 by PCR process, respectively and cloning them into PET-28a vector, transforming E. coli, inducing expression via IPTG, and purifying with a Ni²⁺ affinity resin.

### Preparation example 2

### The preparation of native TRAP proteins

The polyclonal antibodies were prepared with recombinant TRAP proteins. Recombinant TRAP proteins were coupled with Sepherose 4B activated by CNBr to prepare purified antibodies with affinity column. The purified native TRAP proteins having a purity of higher than 95% were obtained by coupling the purified antibodies with Sepherose4B activated by CNBr, purifying TRAP proteins from sonicated supernatant of *Staphylococcus aureus RN6390B* by affinity purification and further purifying by HPLC.

### Preparation example 3

### The preparation of a fusion protein consisting of a TRAP protein and a polypeptide of 12 amino acids

A fusion protein consisting of a TRAP protein and a polypeptide of 12 amino acids was prepared by selecting an unrelated peptide sequence (TPSIPSLWWTTP, Shen Tong et al., Journal of Cellular and Molecular Immunology, 2002, 18: 623-626), designing primes, obtaining a gene sequence expressing a fusion protein of TRAP-untelated peptide by PCR, cloning it into pET-28a vector, inducing expression via IPTG, transforming E. coli, and purifying with Ni²⁺affinity resin.

### Example 1

### The detection of effects of TRAP proteins from multiple origins on Staphylococcus aureus exotoxins in MDBK cell model by MTT method (Normano G. et al, 2001, Microbiological, 24, 341-346)

The concrete steps were as follows:
(1) culturing *Staphylococcus aureus* at 37°C for overnight; dissolving various proteins prepared and purified in preparation examples 1, 2 or 3 into physiological saline, respectively, to obtain a solution with a final concentration of 2µg/µl;
(2) centrifugating the overnight cultured *Staphylococcus aureus* at 5000 rpm/5min to collect bacteria, re-suspending and diluting with THB media (Todd Hewitt Broth, Difico Corporation) to OD₆₀₀nm = 8, inoculating in 0.9 ml THB media at a ratio of 1:100;
(3) adding 100 µl of TRAP protein prepared in step (1) to the media inoculated with *Staphylococcus aureus* obtained in step (2), using 100 µl of physiological saline as a control, and incubating at 37°C for 6 hours;
(4) contrifugating at 10000 rpm/5min to collect supernatant, boiling for 10 minutes, and contrifugating at 10000 rpm/5min to collect supernatant again;
(5) seeding MDBK cell in a 96-well plate (wherein the culture solution is DMEM comprising 10% of fetal bovine serum) at 1 x 104/well, conducting adherent culture at 37°C for 4 hours in a CO₂ incubator;
(6) adding the supernatant obtained from step (4) to the MDBK culture liquid (10µl/well), and culturing at 37°C for 24 hours in a CO₂ incubator; and
(7) detecting the survival status of cells by MTT.

In Fig. 5, the ordinate represents the survival status of cells, wherein a high OD value means a high survival rate of cells and a low generation rate of *Staphylococcus aureus* exotoxins,

It can be seen from Fig.5 that each group of cells with exogenous TRAP proteins shows a higher survival rate, while that without exogenous TRAP proteins shows a lower survival rate, which demonstrates that the exogenous TRAP proteins can reduce the generation of *Staphylococcus aureus* exotoxins. In addition, there is no significant difference among TRAP proteins from different origins with respect to the effect of inhibiting the generation of *Staphylococcus aureus* exotoxins.

### Example 2

### The defection of therapeutic effect of TRAP proteins on Staphylococcus aureus infections in a peritoneal infection animal model

### Preparation of Materials:

BALB/c mice: bought from Animal Center of the Academy of Military Medical Sciences; and other materials: being the same as those in preparation examples 1 and 3.

The concrete detecting steps are as follows:
(1) culturing *Staphylococcus aureus* at 37°C overnight; dissolving two genetically engineered recombinant proteins prepared and purified in preparation example 1 and the TRAP fusion protein prepared and purified in preparation example 3 into physiological saline, respectively, to obtain a solution with a final concentration of 2µg/µl;
(2) centrifugating the overnight cultured *Staphylococcus aureus* in step (1) at 5000 rpm/5min to collect bacteria, re-suspending and diluting with THB media(Todd Hewitt Broth, Difico Corporation) to OD₆₀₀ₙₘ = 8, inoculating in 1 ml THB media at a ratio of 1:100, culturing at 37°C to OD₆₀₀ₙₘ = 5, contrifugating to collect bacteria, and re-suspending with an equal-volume physiological saline;
(3) grouping BALB/c mice (20-25g, male) to 4 groups, wherein each group has 20 mice;
(4) peritoneally injecting 100µl of *Staphylococcus aureus* to mice, after 5 hours, peritoneally injecting various TRAP proteins to the mice, and using physiological saline as a control; and
(5) recoding the survival time and status of each group of mice.

The results show that TRPA proteins can effectively reduce *Staphylococcus aureus* infections in mice and increase the survival rate of infected mice (see Table 1).

**Table 1 The therapeutic effect of TRAP protein on Staphylococcus aureus infection**

| test group | Number of Deaths | | | |
|---|---|---|---|---|
| | 1-3 days | 3-5days | 5-7days | survival |
| 1 | 0 | 6 | 0 | 14 |
| 2 | 0 | 5 | 0 | 15 |
| 3 | 0 | 7 | 1 | 12 |
| 4 | 5 | 13 | 0 | 2 |

| | | | | |
|---|---|---|---|---|
| Note: group 1 represents the group treated with the TRAP protein (GenBank ^{™}Accession No.: AF202641); group 2 represents the group treated with the TRAP protein (GenBank ^{™} Accession No.: AY248703); Group 3 represents the group treated with the fusion protein consisting of a TRAP protein and a peptide of 12 amino acids; and group 4 represents the control group treated with physiological saline. | | | | |

### Example 3

The effect of multiple injections of TRPA on the production of antibodies in vivo in mice
(1) The materials and methods are the same as those in the preparation examples 1 and 2, and the difference lies in that the TRAP protein (GenBank^{™} Accession No.: AF202641) was administrated to mice for three times by peritoneal injection, once every 7 days. After 1 month, the mouse serum was collected, and the production of antibodies against TRAP in vivo in mouse was detected.
(2) Results: multiple injections of TRAP can induce the production of antibodies against TRAP in vivo in mouse, while there is almost no antibody produced in mouse which didn't undergo such injection, indicating that an exogenous TRAP can induce animals to produce antibodies against TRAP in vivo (see Figure 6).

### References:

1. Balaban et al, Science, 1998, Vol.280: 438-440
2. Naomi B, et al, J. Biol. Chem., 2001, 276: 2658-2667
3. Yang G. et al, J. Biol.-Chem., 2005, 280: 27431-27435
4. Sheng Tong et al, Journal of Cellular and Molecular Immunology, 2002, 18: 623-626

## Claims

1. Use of TRAP per se as an active ingredient in the manufacture of a medicament for the treatment of *Staphylococcus aureus* infections, wherein said TRAP has a sequence identity of at least 70% to the sequence as shown in SEQ ID NO: 2 or 4.

2. The use according to claim 1, wherein said TRAP is selected from the group consisting of native TRAP, recombinant TRAP, intact TRAP, fusion proteins comprising TRAP, chemical modifiers of TRAP, or biologically active fragments thereof.

3. The use according to claim 1, wherein said TRAP is from *Staphylococci.*

4. The use according to claim 3, wherein said TRAP protein is from *Staphylococcus aureus.*

5. The use according to claim 1, wherein said TRAP has a sequence identity of at least 80% to the sequence as shown in SEQ ID NO: 2 or 4.

6. The use according to claim 5, wherein said TRAP has a sequence identity of at least 90% to the sequence as shown in SEQ ID NO: 2 or 4.

7. The use according to claim 6, wherein said TRAP has a sequence identity of at least 95% to the sequence as shown in SEQ ID NO: 2 or 4.

8. The use according to claim 7, wherein said TRAP has a sequence identity of more than 95% to the sequence as shown in SEQ ID NO: 2 or 4.

9. The use according to claim 8, wherein said TRAP has a sequence identity of more than 98% to the sequence as shown in SEQ ID NO: 2 or 4.

10. The use according to claim 9, wherein said TRAP has a sequence identity of 100% to the sequence as shown in SEQ ID NO: 2 or 4.

11. The use according to claim 1, wherein said medicament is a pharmaceutical composition comprising TRAP and a pharmaceutically acceptable carrier.

12. The use according to claim 1, wherein said medicament is present in a dosage form including: sterile aqueous or non-aqueous injectable solutions, dispersions, suspensions or emulsions, sterile powders and freeze-dried powders for parenteral administration; hard or soft capsules, tablets, troches, sugar-coated pills, pills, lozenges, powders and granules for oral administration; emulsions, aqueous or oily suspensions, syrups and elixirs; and suppositories, aerosols, ointments, creams, gelatins, inhalations, pasters for skin and implants for external use.

## Patentansprüche

1. Verwendung von TRAP per se als Wirkstoff bei der Herstellung eines Medikaments zur Behandlung von *Staphylococcus aureus* Infektionen, wobei das TRAP eine Sequenz-Identität von mindestens 70 % gegenüber der Sequenz aufweist, die in SEQ ID NO: 2 oder 4 dargestellt ist.

2. Verwendung nach Anspruch 1, wobei das TRAP ausgewählt ist unter nativem TRAP, rekombinantem TRAP, intaktem TRAP, TRAP enthaltenden Fusionsproteinen, chemisch modifiziertem TRAP oder deren biologisch aktiven Fragmenten.

3. Verwendung nach Anspruch 1, wobei das TRAP aus *Staphylococcen* stammt.

4. Verwendung nach Anspruch 3, wobei das TRAP Protein aus *Staphylococcus aureus* stammt.

5. Verwendung nach Anspruch 1, wobei das TRAP eine Sequenz-Identität von mindestens 80 % gegenüber der Sequenz aufweist, die in SEQ ID NO: 2 oder 4 dargestellt ist.

6. Verwendung nach Anspruch 5, wobei das TRAP eine Sequenz-Identität von mindestens 90 % gegenüber der Sequenz aufweist, die in SEQ ID NO: 2 oder 4 dargestellt ist.

7. Verwendung nach Anspruch 6, wobei das TRAP eine Sequenz-Identität von mindestens 95 % gegenüber der Sequenz aufweist, die in SEQ ID NO: 2 oder 4 dargestellt ist.

8. Verwendung nach Anspruch 7, wobei das TRAP eine Sequenz-Identität von mehr als 95 % gegenüber der Sequenz aufweist, die in SEQ ID NO: 2 oder 4 dargestellt ist.

9. Verwendung nach Anspruch 8, wobei das TRAP eine Sequenz-Identität von mehr als 98 % gegenüber der Sequenz aufweist, die in SEQ ID NO: 2 oder 4 dargestellt ist.

10. Verwendung nach Anspruch 9, wobei das TRAP eine Sequenz-Identität von 100 % gegenüber der Sequenz aufweist, die in SEQ ID NO: 2 oder 4 dargestellt ist.

11. Verwendung nach Anspruch 1, wobei das Medikament eine pharmazeutische Zusammensetzung ist, die TRAP und einen pharmazeutisch akzeptablen Träger umfasst.

12. Verwendung nach Anspruch 1, wobei das Medikament in einer Dosierungsform vorliegt, die sterile wässrige oder nicht wässrige injizierbare Lösungen, Dispersionen, Suspensionen oder Emulsionen, sterile Pulver und gefriergetrocknete Pulver zur parenteralen Verabreichung; Hart- oder Weichkapseln, Tabletten, Pastillen, zuckerbeschichtete Dragees, Dragees, Lutschtabletten, Pulver und Granulate zur oralen Verabreichung; Emulsionen, wässrige oder ölige Suspensionen, Dicksäfte und Elixiere; und Suppositorien, Aerosole, Salben, Cremes, Gelatinepräparate, Inhalationen, Hautpasten und Implantate zur externen Anwendung einschließt.

## Revendications

1. Utilisation de TRAP per se en tant qu'ingrédient actif dans la fabrication d'un médicament destiné au traitement d'infections à *Staphylococcus aureus,* où ladite TRAP présente une identité de séquence d'au moins 70 % avec la séquence telle que montrée dans SEQ ID NO: 2 ou 4.

2. Utilisation selon la revendication 1, dans laquelle ladite TRAP est sélectionnée dans le groupe consistant en une TRAP native, une TRAP recombinante, une TRAP intacte, des protéines de fusion comprenant TRAP, des modificateurs chimiques de TRAP, ou des fragments biologiquement actifs de ceux-ci.

3. Utilisation selon la revendication 1, dans laquelle ladite TRAP est issue de *Staphylococci.*

4. Utilisation selon la revendication 3, dans laquelle ladite protéine TRAP est issue de *Staphylococcus aureus.*

5. Utilisation selon la revendication 1, dans laquelle ladite TRAP présente une identité de séquence d'au moins 80 % avec la séquence telle que montrée dans SEQ ID NO: 2 ou 4.

6. Utilisation selon la revendication 5, dans laquelle ladite TRAP présente une identité de séquence d'au moins 90 % avec la séquence telle que montrée dans SEQ ID NO: 2 ou 4.

7. Utilisation selon la revendication 6, dans laquelle ladite TRAP présente une identité de séquence d'au moins 95 % avec la séquence telle que montrée dans SEQ ID NO: 2 ou 4.

8. Utilisation selon la revendication 7, dans laquelle ladite TRAP présente une identité de séquence de plus de 95 % avec la séquence telle que montrée dans SEQ ID NO: 2 ou 4.

9. Utilisation selon la revendication 8, dans laquelle ladite TRAP présente une identité de séquence de plus de 98 % avec la séquence telle que montrée dans SEQ ID NO: 2 ou 4.

10. Utilisation selon la revendication 9, dans laquelle ladite TRAP présente une identité de séquence de 100 % avec la séquence telle que montrée dans SEQ ID NO: 2 ou 4.

11. Utilisation selon la revendication 1, dans laquelle ledit médicament est une composition pharmaceutique comprenant TRAP et un véhicule pharmaceutiquement acceptable.

12. Utilisation selon la revendication 1, dans laquelle ledit médicament est présent sous une forme posologique comprenant : des solutions injectables stériles aqueuses ou non aqueuses, des dispersions, des suspensions ou des émulsions, des poudres stériles et des poudres lyophilisées pour une administration parentérale ; des capsules dures ou molles, des comprimés, des tablettes, des pilules dragéifiées, des pilules, des pastilles, des poudres et des granules pour une administration orale ; des émulsions, des suspensions aqueuses ou huileuses, des sirops et des élixirs ; et des suppositoires, des aérosols, des pommades, des crèmes, des gélatines, des inhalations, des emplâtres pour la peau et des implants pour un usage externe.
